# EUROPEAN PATENT APPLICATION

(11) **EP 1 386 633 A1**
(43) Date of publication of application: **04.02.2004**
(21) Application number: 02425512.7
(22) Date of filing: 02.08.2002
(51) Int. Cl.: A61M 25/06

(54) **Safety catheter**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A safety catheter (1) comprises a catheter body (10) having an axial channel (11) communicating with a sheath or cannula (20) for administration of fluids, a guide needle (3) insertable, though the channel (11) of the catheter body, into the sheath (20) to guide it during insertion into a patient's body (10), and a guide needle body (30), supporting the guide needle (3), insertable into the catheter body (10) and provided with at least one seat (35) for coupling with medical instruments, the body (30) of the guide needle being mounted slidably in said catheter body (10) to be able to pass from a forward working position, wherein the guide needle (3) protrudes forward from the catheter body, to a retracted safety position, wherein the guide needle (3) is protected inside the catheter body (10), there being provided locking means (40) disposed in the body (30) of the guide needle, cooperating with complementary locking or stop means (14, 15, 16) disposed in the catheter body (10), to lock the guide needle body (30) respectively in the forward working position and in the retracted safety position.

## Description

The present invention refers to a safety catheter of the type provided with a guide needle or cannula needle for injection and administration of medicinal fluids into a cavity of a patient's body.

As is known, catheters serve not only for drainage of physiological fluids from the body, but also for injection and administration of medicinal fluids into the patient's body. For this purpose, a catheter generally comprises a central body connected to a tube or flexible sheath, commonly known as a cannula, designed to be inserted into the patient's body for administration of medicinal fluids. Connectors are provided in the body of the catheter for connection of the catheter to bottles of medicinal fluids or to other medical instruments, such as pumps for the administration of and therapy with medicinal fluids.

The boy of the catheter also has an aperture with an axial channel wherethrough the guide needle which enters the catheter sheath to guide it inside the patient's body is inserted. Once the catheter has been used, the needle tip remains outside the body of the catheter or the entire guide needle is extracted from the catheter body to be sent for disposal.

It is obvious that this operation of extraction of the guide needle is hazardous, with the risk of injury to the user or to the personnel responsible for disposal. Furthermore, if the guide needle remains inserted in the catheter, it must be considered that the tip of the guide needle protrudes forward from the catheter body and in any case this leads to the risk of injury or accidental needle sticks.

The object of the present invention is to eliminate the drawbacks of the prior art by providing a safety catheter that is able to avoid the danger and risk of accidental needle sticks.

Another object of the present invention is to provide such a safety catheter that is practical and simple for the user to use.

Yet another object of the present invention is to provide such a safety catheter that is versatile and able to be applied to different types of commercially available medical accessories.

Yet another object of the present invention is to provide such a safety catheter that is composed of few elements and at the same time is inexpensive and simple to make.

These objects are achieved in accordance with the invention with the characteristics listed in appended independent claim 1.

Advantageous embodiments of the invention are apparent from the dependent claims.

The safety catheter according to the invention comprises a catheter body having an axial channel communicating with a sheath or cannula for administration of fluids. A guide needle is mounted on a guide needle body. Said guide needle can be inserted, through the channel in the catheter body, into the sheath to guide it during insertion into the patient's body. The guide needle body is provided with at least one seat for coupling to medical instruments.

The main characteristic of the invention is represented by the fact that the guide needle body is mounted slidably in said catheter body so as to be able to pass from a forward working position, wherein the guide needle protrudes forward from the catheter body, to a retracted safety position, wherein the guide needle is protected inside the catheter body.

The safety catheter according to the invention further comprises locking means disposed in the guide needle body, cooperating with complementary locking or stop means disposed in the catheter body, to lock the guide needle body respectively in the forward position of use and in the retracted safety position.

The advantages of the safety catheter according to the invention are obvious. In fact, once the administration of medicinal fluids has been performed, said safety catheter allows the guide needle to be protected within the body of the catheter, thereby avoiding the risk of accidental needle sticks by the user.

Further characteristics of the invention will be made clearer by the detailed description that follows, referring to a purely exemplary and therefore non-limiting embodiment thereof, illustrated in the appended drawings, wherein:
Figure 1 is a perspective view, illustrating the safety catheter of the invention exploded;
Figure 2 is an axial sectional view of the safety catheter of Figure 1, assembled and in position ready for use, wherein the guide needle and the sheath are partially broken off;
Figure 3 is an enlarged view of a detail of Figure 2, wherein the elastic tongue of the operating pin is shown bent over;
Figure 4 is an axial sectional view illustrating a detail of the safety catheter, wherein the guide needle is in its retracted safety position; and
Figure 5 is a perspective view of the safety catheter, assembled with the guide needle in its retracted safety position, wherein the sheath of the guide needle has been omitted.

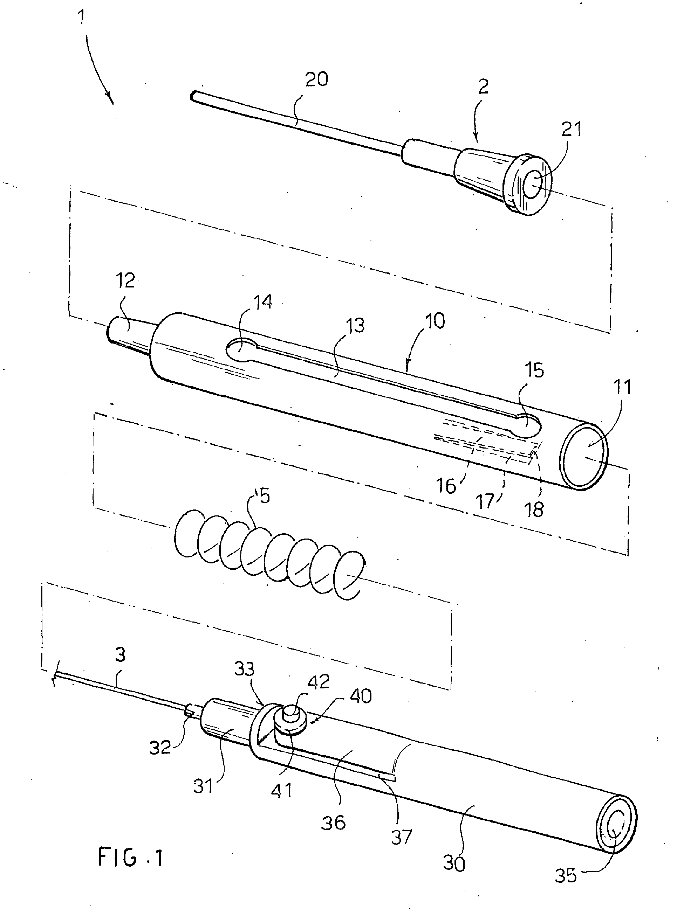

The safety catheter according to the invention will be described with the aid of the figures. With reference in particular to Figure 1, a catheter, indicated as a whole with reference numeral 1, comprises a substantially cylindrical body 10, hollow on the inside so as to define a channel or axial chamber 11 open at the front and rear.

The body 10 of the catheter has a head 12 with a smaller diameter and a substantially truncated conical shape. A support 2 supporting a sheath or cannula 20, in the form of a small flexible tube, hollow on the inside, is mounted on the head 12. The support 2 is hollow on the inside and has an axial channel 21 able to receive he head 12 in a conical coupling relationship.

A longitudinal slot or groove 13, substantially rectangular in shape, is formed in the catheter body 10 and extends for a good part of the length of the catheter body 10. The longitudinal groove 13 has at its front and rear ends two substantially circular stop seats 14 and 15. The diameter of the front 14 and rear 15 stop seats is slightly greater than the width of the longitudinal groove 13.

A longitudinal flexible tongue 16, shown with a dashed line in Figure 1, is formed in the body 10 of the catheter, in a position diametrically opposite the longitudinal groove 13. The longitudinal tongue 16 is formed by means of a substantially U-shaped cut 17 made on the catheter body 10, so as to be able to bend elastically toward the inside and toward the outside of the catheter body. The longitudinal tongue 16 has an abutment and stopping surface 18 facing toward the rear end of the catheter body. Under normal conditions the abutment end 18 of the tongue 16 is situated inside the catheter body.

A guide needle 3 is mounted in the head 32 of a body 30 of the guide needle. The body 30 of the guide needle is substantially cylindrical in shape, with an outside diameter slightly smaller than the inside diameter of the catheter body 10, so as to be able to slide axially in the channel 11 of the catheter body 10.

The body 30 of the guide needle has at the front a cylindrical tang 31 with a smaller diameter than the body, so as to define an annular abutment surface 33 at the front end of the body 30. A head 32 having a smaller diameter than the tang 31 is formed on the cylindrical tang 31 of the body of the guide needle. The head 32 supports the guide needle so that said guide needle 3 protrudes forward and axially therefrom

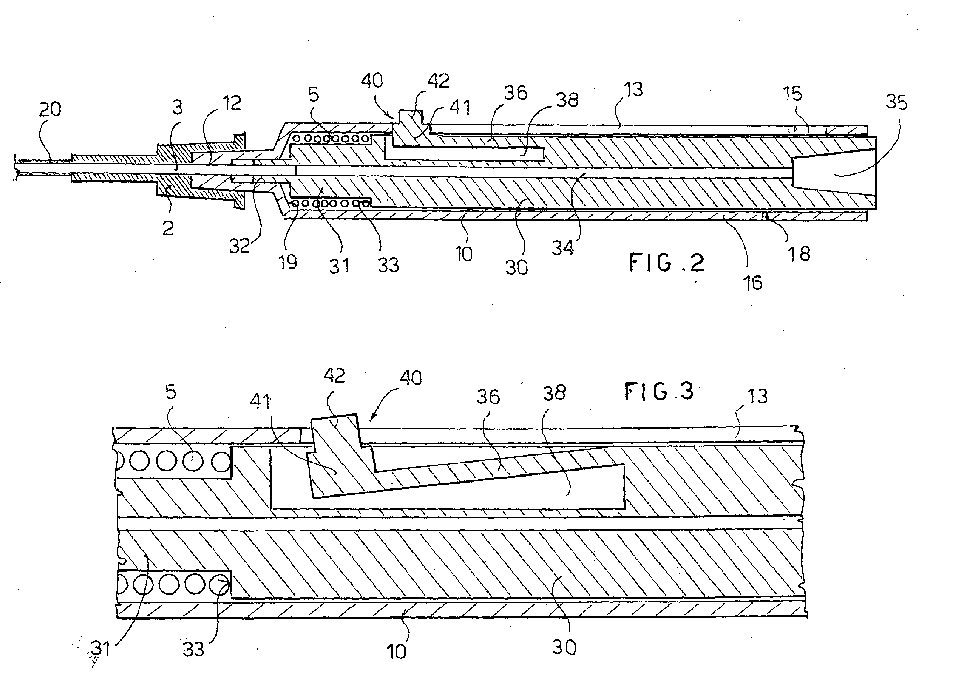

As shown in Figure 2, the body 30 of the guide needle has a channel 34 axially that communicates at the front with the guide needle 3 and at the rear with a conical seat 35. The conical seat 35 is open at the rear to receive, in a conical coupling relationship, the head of medical instruments for the administration of treatment.

A longitudinal elastic tongue 36 is formed in the front part of the body 30 of the guide needle. The longitudinal elastic tongue 36 is defined by a substantially U-shaped cut 37 made in the body 30 of the guide needle.

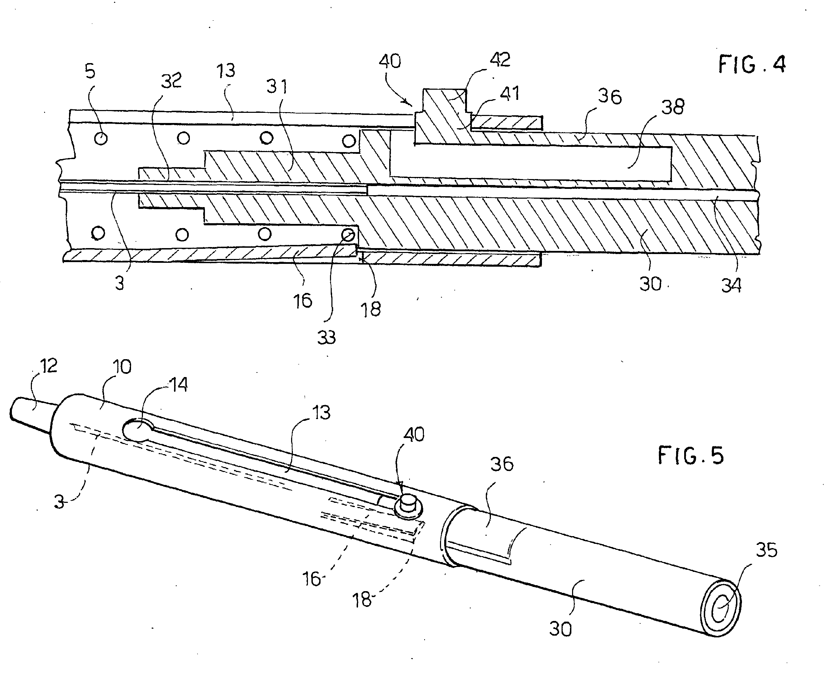

As shown in Figure 2, a longitudinal seat 38 not communicating with the axial channel 34 of the body 30 is formed in the body 30 of the guide needle, below the longitudinal tongue 36. In this manner, as shown in figure 3, the longitudinal tongue 36 can bend elastically in the seat 38 and then return to the starting position illustrated in Figure 2.

A pin 40 protruding radially outward is provided on the elastic tongue 36, in proximity to the free end thereof. The pin 40 has a base part 41, substantially cylindrical in shape, and a top part 42, substantially cylindrical in shape and having a smaller diameter than the base part 41.

The larger diameter base part 41 of the pin 40 has a diameter slightly smaller than the diameter of the front 14 and rear 15 stop seats of the catheter body and slightly greater than the width of the longitudinal slot 13 of the catheter body. The smaller diameter top part 42 of the pin 40, on the other hand, has a diameter slightly smaller than the width of the longitudinal slot 13.

In this manner the base part 41 of the pin 40 can be housed and retained in the front 14 or rear 15 stop seat of the catheter body. Whereas when the elastic tongue 36 is bent inward, the upper wall 42 of the pin can slide guidedly in the longitudinal slot 13 of the catheter body.

Although the drawings illustrate only that the pin 40 is disposed on an elastic tongue 36 defined by the U-shaped cut 37 and bendable in the seat 38, for operation of the safety catheter according to the invention provision can be made for the pin 40 to be mounted on any element that can be elastically compressed by the user and is able to return elastically to the starting position.

Lastly, the safety catheter 1 comprises a spiral spring 5 able to be disposed around the tang 31 of the body of the guide needle. In this manner, as shown in Figure 2, one end of the spring 5 abuts in the radial abutment surface 33 of the body 30 of the guide needle and the other end of the spring abuts on a radial abutment surface 19 defined at the front end of the catheter body 10.

Assembly and operation of the safety catheter 1 according to the invention are described hereunder by way of example.

The body 30 of the guide needle together with the spring 5 disposed around the tang 31 is inserted from the rear into the channel 11 of the catheter body 10. During this insertion the elastic tongue 36 of the guide needle body is bent inwards and the smaller diameter top part 42 of the pin 40 slides guidedly in the longitudinal slot 13 of the catheter body, until the pin 40 reaches the front stop seat 14.

At this point the elastic tongue 36 of the guide needle body returns elastically to the starting position and the larger diameter base part 41 of the pin 40 is housed in the front stop seat 14. In this situation, as shown in Figure 2, the spring 5 is compressed between the abutment surface 33 of the guide needle body and the abutment surface 19 of the catheter body and the guide needle 3 is in its working position, extracted from the head 12 of the catheter body. At this point the support 2 with the relative cannula can be applied to the head 12 of the catheter body (as shown in Figure 2).

Once treatment has been completed, the operator manually presses the pin 40. Consequently, the larger diameter base part 41 of the pin 40 disengages from the front stop seat 14 of the slot 13 of the catheter body and the spring 5 which was compressed is released, axially biasing the catheter body 10 and the guide needle body 30 in opposite directions. As a result the guide needle 3 together with its body 30 retracts with respect to the catheter body 10 and therefore the guide needle 3 is disposed in its safety position, illustrated in Figure 5, wherein it is protected by the catheter body 10.

It should be noted that the catheter body 30 is guided during the withdrawal stroke since the smaller diameter top part 42 of the pin 40 slides guidedly in the slot 13 of the catheter body until the pin reaches the top stop seat 15. At this point the elastic tongue 36 of the catheter body returns elastically to its starting position and the larger diameter base part 41 of the pin 40 is housed in the rear stop seat 15, preventing any axial movement of the guide needle body 30 with respect to the catheter body.

As shown in Figure 4, in this situation the longitudinal tongue 16 of the catheter body, which acts as a further safety element, also intervenes. In fact said tongue 16 returns elastically to its inwardly bent position and its abutment surface 18 abuts against the abutment surface 33 of the front end of the guide needle body.

Numerous variations and changes of detail within the reach of a person skilled in the art can be made to the present embodiment of the invention, without departing from the scope of the invention as set forth in the appended claims.

## Claims

1. A safety catheter (1) comprising:
- a catheter body (10) having an axial channel (11) communicating with a sheath or cannula (20) for administration of fluids,
- a guide needle (3) insertable through the channel (11) of the catheter body, into the sheath (20) to guide it during insertion into the patient's body, and
- a guide needle body (30) supporting said guide needle (3), insertable inside the catheter body (10) and provided with at least one seat (35) for coupling with medical instruments,
**characterised in that**
said body (30) of the guide needle is mounted slidably inside the catheter body (10) to be able to slide from a forward working position wherein the guide needle (3) protrudes forward from the catheter body to a retracted safety position wherein the guide needle (3) is protected inside the catheter body (10), there being provided locking means (40) disposed in said body (30) of the guide needle, cooperating with complementary locking or stop means (14, 15, 16) disposed in the catheter body (10), to lock the body (30) of the guide needle respectively in said forward working position and in said retracted safety position.

2. A safety catheter (1) according to claim 1, **characterised in that** said locking means provided in the guide needle body (30) comprise a pin (40) and said complementary locking means provided in the catheter body (10) comprise two seats (14, 15) disposed at the front and at the rear in the catheter body to receive said pin (40) when the body (30) of the guide needle is situated respectively in said forward working position and in said retracted safety position.

3. A safety catheter (1) according to claim 2, **characterised in that** said pin (40) is mounted on an elastic element (36) connected to the guide needle body (10), so as to be able to be squeezed manually by the operator, causing elastic yielding of said elastic element (36), to disengage itself from said front seat (14) of the catheter body, when the guide needle body (30) is in said forward working position.

4. A safety catheter (1) according to claim 3, **characterised in** the tat said elastic element (36) supporting the pin (40) consists of an elastic tongue (36) defined by a substantially U-shaped cut (37) in said guide needle body (30) and disposed in a longitudinal seat (38) formed in the guide needle body (30), so as to be able to bend in said longitudinal seat (38).

5. A safety catheter (1) according to any one of the preceding claims, **characterised in that** it comprises guide means (13) able to guide the axial sliding of said guide needle body (30) inside said catheter body (10).

6. A safety catheter (1) according to claim 5, **characterised in that** said guide means comprise a longitudinal slot (13) formed in the catheter body (10) and ending in said front (14) and rear (15) seats to allow guided sliding of said pin (40) in said longitudinal slot (13).

7. A safety catheter (1) according to claim 6, **characterised in that** said front and rear seats (14, 15) are substantially circular in shape with a slightly larger diameter than the width of said longitudinal slot (13), and
said pin (40) comprises a cylindrical base part (41) having a larger diameter than the upper cylindrical part (42),
the diameter of said base part (41) of the pin being slightly smaller than the diameter of said front and rear seats (14, 15) and slightly greater that the width of said longitudinal slot (13), and
the diameter of said top part (42) of the pin being slightly smaller than the width of said longitudinal slot (13).

8. A safety catheter (1) according to any one of the preceding claims, **characterised in that** said complementary locking or stop means (16) disposed in the body (10) of the catheter further comprise a longitudinal elastic tongue (16) disposed in the rear part of the catheter body (10), said longitudinal elastic tongue (16) being defined by a substantially U-shaped cut (17) formed in the catheter body and having a free end (18) protruding inward to abut against an abutment surface (33) formed in the front part of the body (30) of the guide needle, when the guide needle body is in its retracted safety position.

9. A safety catheter (1) according to any one of the preceding claims, **characterised in that** it comprises spring means (5) disposed in the catheter body (10) between an abutment surface (19) provided in the front wall of the guide needle body (30), said spring means (5) being under compression, when the guide needle body (30) is in said forward working position and being released when the guide needle body (30) is in the retracted safety position.

10. A safety catheter (1) according to claim 9, **characterised in that** said spring means comprise a spiral spring (5) disposed around a cylindrical tang (31) disposed in front of said guide needle body (30), so as to define said abutment surface (33) of the guide needle body and having a smaller diameter than the guide needle body.
